# EUROPEAN PATENT APPLICATION

(11) **EP 4 564 355 A1**
(43) Date of publication of application: **04.06.2025**
(21) Application number: 23847046.2
(22) Date of filing: 28.07.2023
(51) Int. Cl.: G16B 45/00, G16B 25/20, G16B 30/00

(54) **APPARATUS AND METHOD FOR PROVIDING INTERFACE FOR SETTING THRESHOLD VALUE OF PARAMETER**

(30) Priority: 29.07.2022 KR 20220094697
(71) Applicant: Seegene, Inc., Seoul 05548 (KR)
(72) Inventor: SON, Hyung Suk, Seoul 05544 (KR); KIM, Hyoung Gyu, Seoul 05545 (KR); KIM, Ji Hye, Seoul 05382 (KR); PARK, Bong Gyun, Seoul 06069 (KR); JUNG, Hwa Young, Seoul 02810 (KR); LEE, Ye Ji, Seoul 08515 (KR); CHA, Kwang Su, Gimpo-si Gyeonggi-do 10068 (KR); LIM, Jeong Min, Seoul 05841 (KR); LYU, Won Woo, Seoul 05401 (KR)
(74) Representative: Viering, Jentschura & Partner mbB Patent- und Rechtsanwälte
(86) International application number: PCT/KR2023/011069
(87) International publication number: WO 2024/025390

(57) **Abstract**

A method for providing an interface for setting positive/negative determination criteria for amplification characteristic parameters according to one embodiment of the present disclosure, includes: obtaining measurement values for n types of amplification characteristic parameters for each of a plurality of samples from detection data obtained in a detection process for a target analyte in each of the plurality of samples, wherein n is an integer of 2 or more and each of the plurality of samples is pre-labeled as either a positive sample or a negative sample; displaying each of the plurality of samples at a position determined by the n types of measurement values in an n-dimensional coordinate system space defined by axes corresponding to the n types of amplification characteristic parameters; indicating whether each of the plurality of samples displayed in the n-dimensional coordinate system space is positive or negative using the pre-labeled results; and displaying a screen for setting a threshold for each of the n types of amplification characteristic parameters.

## Description

### TECHNICAL FIELD

The present disclosure relates to a computer-implemented method, system and storage medium for setting thresholds of parameters.

### BACKGROUND ART

Target analytes or target substances, particularly target nucleic acid molecules, can be amplified using various methods. The various methods of amplification may include the following: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker et al. Nucleic Acids Res. 20(7):1691-6 (1992); Walker PCR Methods Appl 3(1):1-6 (1993)), transcription-mediated amplification (Phyffer et al., J. Clin. Microbiol. 34:834-841 (1996); Vuorinen et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)), Q-Beta Replicase (Lizardi et al., BiolTechnology 6:1197(1988)), loop-mediated isothermal amplication (LAMP, Y. Mori, H. Kanda and T. Notomi, J. Infect. Chemother, 2013, 19, 404-411), and recombinase polymerase amplication (RPA, J. Li, J. Macdonald and F. von Stetten, Analyst, 2018, 144, 31-67).

In nucleic acid amplification reactions, the real-time PCR (Real-time PCR) system is a technology for detecting target nucleic acids in a sample in real time. In such a real-time PCR system, in order to detect a specific target nucleic acid, a signal generating means that emits a detectable fluorescent signal in proport'ion to the amount of the target nucleic acid is used. Specifically, a fluorescent signal generated by the signal generating means in proportion to the amount of the target nucleic acid is detected for each cycle. A data set including each measurement point and the intensity of the fluorescent signal at the measurement point is obtained. From the data set obtained in this way, an amplification curve or amplification profile curve that indicates the intensity of the fluorescent signal detected at each measurement point is obtained.

Based on the aforementioned data set, amplification curve or amplification profile curve, a process for determining the positivity or negativity of a specific target nucleic acid in a sample is performed. Specifically, the values of parameters for the aforementioned amplification curve or amplification profile curve may be obtained for each target nucleic acid. The obtained values of the parameters can be compared with criteria determined in advance for positive/negative determination to determine the positivity or negativity. In other words, in order to determine the positivity or negativity, a criterion for the positivity or negativity needs to be determined in advance, and the method for determining the criterion may affect the accuracy and efficiency of target analysis.

### DETAILED DESCRIPTION OF INVENTION

### TECHNICAL PROBLEMS

The objectives to be achieved in one embodiment include providing an interface used to set a value of a criterion for determining positive or negative of a target analyte in a sample as described above.

Meanwhile, in the process of determining positive or negative of the target analyte in the sample, a correction operation for the aforementioned data set, amplification curve, or amplification profile curve may be performed. In the correction operation, pre-determined values of parameters may be used. The pre-determined values of parameters also affect the results of the aforementioned positive/negative determination process.

Accordingly, an additional objective of one embodiment includes providing an interface used to set values of parameters used in the aforementioned correction operation.

However, the objectives to be achieved by the present disclosure are not limited to those mentioned above, and other objectives that are not mentioned will be clearly understood by a person having ordinary skill in the art to which the present disclosure pertains from the description below.

### TECHNICAL SOLUTION

A method for providing an interface for setting positive/negative determination criteria for amplification characteristic parameters according to one embodiment, includes: obtaining measurement values for n types of amplification characteristic parameters for each of a plurality of samples from detection data obtained in a detection process for a target analyte in each of the plurality of samples, wherein n is an integer of 2 or more and each of the plurality of samples is pre-labeled as either a positive sample or a negative sample; displaying each of the plurality of samples at a position determined by the n types of measurement values in an n-dimensional coordinate system space defined by axes corresponding to the n types of amplification characteristic parameters; indicating whether each of the plurality of samples displayed in the n-dimensional coordinate system space is positive or negative using the pre-labeled results; and displaying a screen for setting a threshold value for at least one of the n types of amplification characteristic parameters, wherein the threshold value is used as a criterion in a positive/negative determination process for each of the plurality of samples.

Further, the screen for setting the threshold value is displayed simultaneously with the n-dimensional coordinate system space where each of the plurality of samples is labeled as positive or negative
Further, the detection process is performed by nucleic acid amplification reaction, enzymatic reaction, or microbial growth.

Further, the n types of amplification characteristic parameters include at least one of a matching parameter indicating a degree of matching between the detection data and an approximation function approximating the detection data, a graph shape parameter representing a graph shape of the approximation function, and a derived parameter derived from the graph shape parameter.

Further, the n types of amplification characteristic parameters include at least one of a maximum value of the first derivative of the approximation function, a difference between a maximum signal value and a minimum signal value of the approximation function, or a sharpness of the approximation function.

Further, in the positive/negative determination process, a measurement value of at least one of the n types of amplification characteristic parameters and the threshold value corresponding thereto are compared.

Further, in the n-dimensional coordinate system space, the axes corresponding to the n types of amplification characteristic parameters are orthogonal to each other.

Further, in the indicating whether each of the plurality of samples is positive or negative, the positive sample and the negative sample are indicated using different colors and/or shapes

Further, at least one of the parameters corresponding to the axes of the coordinate system is selected by a user.

Further, the screen for setting the threshold value is provided with a slide bar that is implemented to be adjustable on the screen, the threshold value is determined by adjusting the corresponding slide bar, and the threshold value determined each time the slide bar is adjusted is reflected and displayed in real time in the n-dimensional coordinate system space
Further, the threshold value is represented as a line or plane in the coordinate system space.

Further, the line or plane is used to group the plurality of samples displayed in the coordinate system space into a positive sample group and a negative sample group
Further, the threshold value includes an upper threshold value and a lower threshold value.

Further, the method further includes indicating at least one of the samples located between the upper threshold value and the lower threshold value as a third state other than the pre-labeled results.

Further, a device for providing an interface for setting positive/negative determination criteria for amplification characteristic parameters according to one embodiment, includes: a memory storing at least one instruction; and a processor, wherein the processor executes the at least one instruction to perform: obtaining measurement values for n types of amplification characteristic parameters for each of a plurality of samples from detection data obtained in a detection process for a target analyte in each of the plurality of samples, wherein n is an integer of 2 or more and each of the plurality of samples is pre-labeled as either a positive sample or a negative sample; displaying each of the plurality of samples at a position determined by the n types of measurement values in an n-dimensional coordinate system space formed by axes corresponding to the n types of amplification characteristic parameters; indicating whether each of the plurality of samples displayed in the n-dimensional coordinate system space is positive or negative using the pre-labeled results; and displaying a screen for setting a threshold value for at least one of the n types of amplification characteristic parameters, wherein each of the n types of threshold values is used as a criterion in a positive/negative determination process for each of the plurality of samples

### EFFECT OF INVENTION

According to one embodiment, an interface can be provided that allows for visual confirmation of an amplification characteristic parameter that can be used as a criterion for determining positive or negative in the positive/negative determination process. In addition, an interface can be provided that effectively performs setting of a positive-negative distribution and boundary values that can be used in the positive/negative determination process by displaying the distribution of amplification characteristic parameters for each of a plurality of samples on a coordinate system.

### BRIEF DESCRIPTION OF THE DRAWINGS

FIG. 1 illustrates a detection device and a parameter threshold-setting interface providing device connected thereto, according to one embodiment.
FIG. 2 is an exemplary block diagram of the parameter threshold-setting interface providing device according to one embodiment.
FIG. 3 illustrates a parameter setting interface for performing a parameter threshold-setting interface providing method according to one embodiment.
FIG. 4 illustrates an enlarged view of a portion of a coordinate system area in FIG. 3.
FIG. 5 is a flowchart of a method for providing an interface for setting a threshold of an amplification characteristic parameter according to one embodiment.

### MODE FOR CARRYING OUT THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

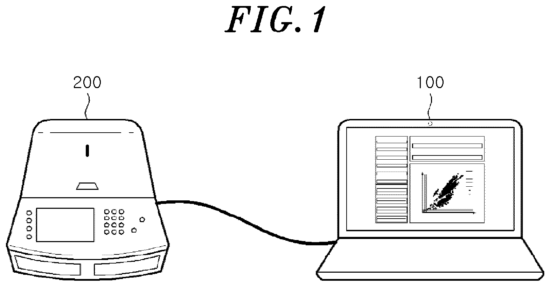

Prior to describing FIG. 1, terms used herein are described.

The term "plate" refers to a standard unit on which an amplification reaction is performed in a PCR device, and means a basic unit on which data generated after the amplification reaction is stored. Different plates may be plates on which an amplification reaction is performed at different times using the same amplification device, or plates on which an amplification reaction is performed at the same time by different amplification devices.

The plate includes a plurality of reaction wells. The plate may include N x M reaction wells. Typically, the plate includes 12 x 8 or 8 x 12 reaction wells. The reaction wells of the plate may be integrated with the plate or may be in the form of a detachable tube. The plate may be rectangular in shape, but the plate may also be implemented in various shapes, such as circular, trapezoidal, and rhomboidal, as long as it includes one or more reaction wells.

The wells of the plate contain samples to be analyzed and reagents necessary for a nucleic acid amplification reaction.

The term "target analyte" includes various substances (e.g., biological substances and non-biological substances), which may refer to the same subject as the term "target analyte substance."

Such target analytes may specifically include biological substances, more specifically at least one of nucleic acid molecules (e.g., DNA and RNA), proteins, peptides, carbohydrates, lipids, amino acids, biological compounds, hormones, antibodies, antigens, metabolites, and cells.

The term "sample" refers to biological samples (e.g., cells, tissues, and body fluids) and non-biological samples (e.g., food, water, and soil). Among these, the biological samples may include at least one of, for example, viruses, bacteria, tissues, cells, blood (including whole blood, plasma, and serum), lymph, bone marrow fluid, saliva, sputum, swab, aspiration, milk, urine, stool, eye fluid, semen, brain extract, spinal fluid, joint fluid, thymic fluid, bronchial lavage fluid, ascites, and amniotic fluid. These samples may or may not contain the target analytes described above.

Meanwhile, when the aforementioned target analyte is a nucleic acid molecule or contains a nucleic acid molecule, a nucleic acid extraction process known in the art can be performed on the sample presumed to include the target analyte (see: Sambrook, J. et al., Molecular Cloning. A Laboratory Manual, 3rd ed. Cold Spring Harbor Press (2001)). The nucleic acid extraction process may vary depending on the type of sample. In addition, when the extracted nucleic acid is RNA, a reverse transcription process for synthesizing cDNA may be additionally performed (see: Sambrook, J. et al., Molecular Cloning. A Laboratory Manual, 3rd ed. Cold Spring Harbor Press (2001)).

The term "data set" refers to data obtained from a signal generation reaction for the target analyte using a signal generation means (which will be described later).

In this case, the term "signal generation reaction" means a reaction that generates a signal dependent on properties of a target analyte in a sample, such as activity, amount or presence (or absence) of the target analyte, specifically presence (or absence). Such signal generation reactions include biological reactions and chemical reactions. Among these, biological reactions include genetic analysis processes such as PCR, real-time PCR, and microarray analysis, immunological analysis processes, and bacterial growth analysis. In addition, chemical reactions include processes that analyze the creation, transformation, or destruction of a chemical substance. According to one embodiment, the signal generation reaction may be a genetic analysis process, or may be a nucleic acid amplification reaction, an enzymatic reaction, or microbial growth.

Meanwhile, the signal generation reaction described above is accompanied by a signal change. Therefore, the progress of the signal generation reaction can be evaluated by measuring the signal change.

In this case, the term "signal" means a measurable output. Furthermore, the measured magnitude or change of the signal serves as an indicator that qualitatively or quantitatively indicates the properties of the target analyte, specifically the presence or absence of the target analyte in the sample.

Examples of the indicator include, but are not limited to, fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectance, transmittance, and absorbance.

The term "signal generating means" as mentioned above refers to a means for providing a signal indicating the properties, specifically the presence or absence, of the target analyte to be analyzed.

Such signal generating means include a label itself or an oligonucleotide to which the label is linked.

Among these, the label includes a fluorescent label, a luminescent label, a chemiluminescent label, an electrochemical label, and a metallic label. The label may be used as is, such as an intercalating dye. Alternatively, the labels may be used in the form of a single label or an interactive dual label comprising a donor molecule and an acceptor molecule, linked to one or more oligonucleotides.

When using a fluorescent label, the signal value may be expressed as RFU (Relative Fluorescence Unit) values.

The signal generating means may additionally include an enzyme having a nucleic acid cleavage activity to generate a signal (e.g., an enzyme having a 5' nucleic acid cleavage activity or an enzyme having a 3' nucleic acid cleavage activity).

Meanwhile, various methods for generating a signal indicating the presence of a target analyte, particularly a target nucleic acid molecule, using the signal generating means are known. Representative examples may include: TaqManTM probe method (U.S. Patent. No. 5,210,015), Molecular beacon method (Tyagi, Nature Biotechnology, v.14 MARCH 1996), Scorpion method (Whitcombe et al., Nature Biotechnology 17:804-807 (1999)), Sunrise or Amplifluor method (Nazarenko et al., Nucleic Acids Research, 25(12):2516-2521 (1997), and U.S. Patent. No. 6,117,635), Lux method (U.S. Patent. No. 7,537,886), CPT (Duck P et al. Biotechniques, 9:142-148 (1990)), LNA method (U.S. Patent. No. 6,977,295), Plexor method (Sherrill CB et al., Journal of the American Chemical Society, 126:4550-4556 (2004)), Hybeacons (D.J. French et al., Molecular and Cellular Probes 13:363-374 (2001) and U.S. Patent. No. 7,348,141), Dual-labeled, self-quenched probe (U.S. Patent. No. 5,876,930), Hybridization probe (Bernard PS et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO 2013/115442), PCE-NH (PTO Cleavage and Extension-Dependent Non-Hybridization) method (PCT/KR2013/012312), and CER method (WO 2011/037306).

Meanwhile, the term "signal generating reaction" mentioned above may refer to an amplification reaction of a signal. In this case, the term "amplification reaction" means a reaction that increases or decreases a signal generated by the signal generation means. Specifically, the amplification reaction means a reaction of increasing (or amplifying) a signal generated by the signal generation means depending on the presence of a target analyte.

In such amplification reactions, amplification of a target analyte (e.g., a nucleic acid molecule) may or may not be accompanied. More specifically, the amplification reaction may mean an amplification reaction of a signal accompanied by amplification of a target analyte.

Meanwhile, the data set obtained through the amplification reaction may include amplification cycles.

Here, the term "cycle" refers to a unit of change in specific conditions in a plurality of measurements involving the change in the specific conditions. The change in the specific conditions may refer to an increase or decrease in temperature, reaction time, number of reactions, concentration, pH, number of replications of a measurement target (e.g., nucleic acid), etc., for example. Accordingly, the cycle may be a time or process cycle, a unit operation cycle, and a reproductive cycle.

More specifically, when a reaction of a certain process is repeated or a reaction is repeated at certain time intervals, the term "cycle" means a single unit of the repetition.

For example, in the case of polymerase chain reaction (PCR), one cycle means a reaction including the denaturation phase of nucleic acid, the annealing phase of primer, and the extension phase of primer. In this case, the change in specific conditions is an increase in the number of repetitions of the reaction, and the repetition unit of the reaction including the series of phases described above is set as one cycle.

Alternatively, when a certain action is repeated as a reaction progresses, the term "cycle" may mean a single unit of the repetition.

For example, when a nucleic acid amplification reaction is performed, the act of detecting a signal generated at regular time intervals may be repeated, and the cycle may mean a single unit of the repetition. In this case, the cycle may have a unit of time. Meanwhile, the amplification reaction for amplifying a signal indicating the presence of a target analyte (or a target analyte substance) may be performed in a manner in which the signal is also amplified while the target analyte is amplified (e.g., real-time PCR method). Alternatively, according to one embodiment, the amplification reaction may be performed in a manner in which only the signal indicating the presence of the target analyte is amplified without amplifying the target analyte (e.g., CPT method (Duck P, et al., Biotechniques, 9:142-148 (1990)), Invader assay (U.S. Patent. Nos. 6,358,691 and 6,194,149)).

Meanwhile, the aforementioned target analytes or target analyte substances, especially target nucleic acid molecules, can be amplified using various methods: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker et al. Nucleic Acids Res. 20(7):1691-6 (1992); Walker PCR Methods Appl 3(1):1-6 (1993)), transcription-mediated amplification (Phyffer et al., J. Clin. Microbiol. 34:834-841 (1996); Vuorinen et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)), Q-Beta Replicase (Lizardi et al., BiolTechnology 6:1197 (1988)), Loop-mediated isothermal amplication (LAMP, Y. Mori, H. Kanda and T. Notomi, J. Infect. Chemother, 2013, 19, 404-411), and Recombinase polymerase amplication (RPA, J. Li, J. Macdonald and F. von Stetten, Analyst, 2018, 144, 31-67).

Meanwhile, the amplification reaction amplifies the signal while amplifying the target analyte (specifically, the target nucleic acid molecule). For example, the amplification reaction is performed using PCR, specifically real-time PCR, or isothermal amplification reaction (e.g., LAMP or RPA).

Meanwhile, the data set obtained by the signal generation reaction includes a plurality of data points including cycles of the signal generation reaction and signal values in the cycles.

In this case, the term "signal value" means a quantified value of a signal level (e.g., signal intensity) actually measured during the cycles of a signal generation reaction, particularly an amplification reaction based on a certain scale, or a transformed value thereof. The transformed value may include a mathematically processed signal value of the actually measured signal value. Examples of mathematically processed signal values of the actually measured signal value (i.e., the signal value of the raw data set) may include logarithmic values or derivatives.

The term "data point" means a coordinate value that includes a cycle and a signal value. In addition, the term "data" means all information that constitutes a data set. For example, each of the cycle and the signal value of an amplification reaction may be considered data.

Data points obtained by a signal generation reaction, particularly an amplification reaction, may be expressed as coordinate values that can be expressed in a two-dimensional Cartesian coordinate system. In the coordinate values, the X-axis represents the number of cycles, and the Y-axis represents the signal value measured or processed in the corresponding cycle.

The term "data set" means a collection of the data points. For example, the data set may be a collection of data points obtained directly through an amplification reaction performed in the presence of a signal generating means, or may be a modified data set obtained by modifying such a data set. The data set may be some or all of a plurality of data points obtained by the amplification reaction, or modified data points thereof.

Meanwhile, the data set may be a data set obtained by processing multiple data sets. When performing analyses on a plurality of target analytes in a single reaction vessel, in some cases, the data sets for the plurality of target analytes may be obtained by processing data sets obtained from reactions performed in the single reaction vessel. For example, the data sets for the plurality of target analytes in the single reaction vessel may be obtained by processing multiple data sets derived from signals measured at different temperatures.

In one embodiment, the reaction vessel provides a closed space in which a reaction is performed. The reaction vessel includes one or more reaction containers. The reaction container refers to a unit that can accommodate a reactant (e.g., a reaction solution or a reaction mixture). Examples of the reaction vessel including one or more reaction containers include a test tube, a PCR tube, a strip tube, and a multi-well PCR plate.

The aforementioned data set may be plotted to obtain an amplification curve.

Hereinafter, various embodiments of the present disclosure will be described with reference to the drawings.

FIG. 1 illustrates a detection device 200 and a parameter threshold-setting interface providing device 100 connected thereto, according to one embodiment. These devices 100 and 200 may be connected to each other by wired or wireless communication. However, the block diagram illustrated in FIG. 1 is merely exemplary, and the idea of the present disclosure is not limited to that illustrated in FIG. 1. For example, a configuration not illustrated in FIG. 1 may be additionally connected to these devices 100 and 200. Alternatively, unlike what is illustrated, the parameter setting interface providing device 100 may be implemented by being included in the detection device 200. However, the following description will be made on the premise that the aforementioned devices 100 and 200 are implemented or connected as illustrated in FIG. 1. Hereinafter, each configuration will be described in detail.

The detection device 200 is implemented to perform a nucleic acid amplification reaction and a nucleic acid detection operation on a sample. Although the detection device 200 may be implemented to perform the nucleic acid detection operation without performing the nucleic acid amplification reaction according to some embodiments, the following description will be made on the premise that the detection device 200 is implemented to perform both the nucleic acid detection operation and the nucleic acid amplification reaction.

Meanwhile, as the aforementioned nucleic acid amplification reaction proceeds, if the sample in the reaction vessel contains a target analyte, not only the amount of the target analyte is amplified, but also the magnitude of the signal generated by the aforementioned signal generating means can be amplified as described above. Accordingly, the detection device 200 is implemented to detect the magnitude of this signal. Specifically, the detection device 200 may monitor in real time the magnitude of the aforementioned signal that changes as the nucleic acid amplification reaction progresses. The monitored results may be output from the detection device 200 in the form of the data set defined above, for example, as the signal intensity per cycle. In this case, the signal intensity per cycle is information as a material that serves as a basis for determining the presence or absence of the target analyte in the corresponding sample.

Meanwhile, since the magnitude of the signal generated by the signal generating means can also be amplified when the nucleic acid amplification reaction is performed, the nucleic acid amplification reaction will be considered hereinafter as causing the signal generating reaction defined above.

The parameter threshold-setting interface providing device 100 provides a user interface for setting parameter values. This will be described in detail below.

Various software may be used in the process of determining the presence or absence of a target analytes in a sample. Among these software, there is a presence/absence determination software that performs the presence/absence determination process for the target analyte in the sample by utilizing the input information and pre-determined parameters when the information as the aforementioned material is input. The performance or accuracy of the presence/absence determination software depends on the value of the corresponding parameter. In addition, the faster the value of the parameter is determined, the quicker the corresponding software and associated reagents can be launched to the market.

Accordingly, the parameter threshold-setting interface providing device 100 according to one embodiment is implemented to provide a user interface for researchers and developers of reagents that enables rapid and accurate determination of such parameter values.

In this case, the aforementioned parameters act as criterions for determining the presence or absence of target analytes in the sample in the presence/absence determination process. There may be various parameters as such criterions, and these will be described later.

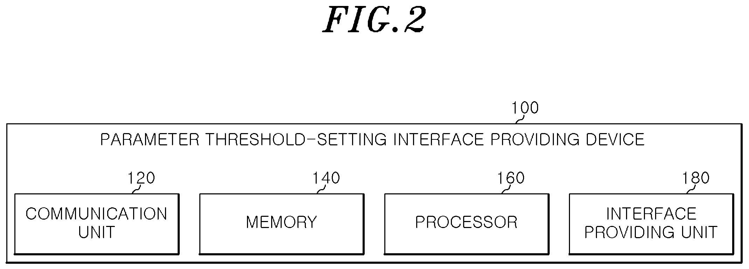

Hereinafter, each functional configuration of the parameter threshold-setting interface providing device 100 will be described in more detail with reference to FIG. 2.

FIG. 2 is an exemplary block diagram of the parameter threshold-setting interface providing device 100 according to one embodiment. Before referring to FIG. 2, it should be noted that the parameter threshold-setting interface providing device 100 can be implemented on a laptop, server, cloud, etc., but the present disclosure is not limited thereto.

Referring to FIG. 2, the parameter threshold-setting interface providing device 100 includes a communication unit 120, a memory 140, a processor 160, and an interface providing unit 180, but the present disclosure is not limited thereto.

First, the communication unit 120 is implemented as a wired or wireless communication module. Through the communication unit 120, the parameter threshold-setting interface providing device 100 can communicate with external systems. For example, the parameter threshold-setting interface providing device 100 may transmit setting values, thresholds, etc. input from a researcher through the communication unit 120, as will be described later. In addition, the parameter threshold-setting interface providing device 100 may receive, through the communication unit 120, pre-labeled correct answer data regarding positive or negative, default values regarding parameters, determination results when the default values are applied to the positive/negative determination process, determination results of the determination process when the setting values or thresholds are applied to the positive/negative determination process, etc.

Various types of data are stored in the memory 140. The stored data may include the correct answer data or default values mentioned above, but the present disclosure is not limited thereto. For example, the memory 140 may store data (or data set) received from the detection device 200 or data processed by the processor 160, which may include the aforementioned data set, a noise-removed data set from which noise has been removed, an amplification curve approximated from the noise-removed data set, parameters extracted from the amplification curve, e.g., a Ct value, signal values (e.g., an RFU value), etc.

In addition, the memory 140 may store data on a plate-by-plate basis or a strip-by-strip basis. Further, the memory 140 may store data for a plurality of plates and amplification data generated based on the data for the plurality of plates.

In this case, the data for the plate includes numbers or characters for identifying the plate, information recorded for the plate, or data for reaction wells included in the plate. Among these, the information recorded for the plate includes various information, such as the date and time when the amplification reaction was performed for the plate, and the method used for the amplification reaction for the plate. In addition, the data for the reaction wells includes a data set for each reaction well, noise-removed data sets, amplification curves, Ct values, signal values, and the like.

Meanwhile, in FIG. 2, the memory 140 is depicted as a separate configuration from the processor 160, but the memory 140 may be implemented as a single device with the processor 160. For example, the memory 140 may be a storage, such as a cache, included inside the processor 160.

Next, the processor 160 may be implemented by a central processing unit (CPU), a graphics processing unit (GPU), a micro controller unit (MCU), or a dedicated processor that performs the methods according to the embodiments. In addition, the processor 160 may be implemented by at least one core.

The processor 160 can write data to the memory 140. In addition, the processor 160 can read out and execute instructions stored in the memory 140. For example, the processor 160 can enable the interface providing unit 180 to perform the functions to be described below by executing instructions stored in the memory 140.

The interface providing unit 180 may be driven by the processor 160 as follows. Before the description, the interface providing unit 180 may be implemented by a means for displaying data and a means for receiving data. For example, the interface providing unit 180 may be implemented by a touch screen or touch pad, or the like, or alternatively, it may be implemented by a combination of an LCD monitor and a keyboard or the like.

First, the interface providing unit 180 may display correct answer data for the presence or absence of a target analyte in a sample. The correct answer data may be externally received by the parameter threshold-setting interface providing device 100 through the communication unit 120 or loaded from the storage unit 140, but the present disclosure is not limited thereto. The correct answer data may be displayed in any coordinate system on the interface so that positive and negative can be distinguished. In this case, the correct answer data is a collection of correct answers where the presence or absence of the target for each sample has been confirmed as positive or negative. If the correct answer (positive or negative) for each reaction vessel receiving the sample is confirmed for each reaction vessel in the entire 96-well plate, it can be referred to as correct answer data for the corresponding 96-well plate. Such correct answer data can be obtained for each well by being labeled by a researcher who manually adjusted the concentration of target nucleic acid for each reaction vessel.

In this case, the target nucleic acid may be artificially synthesized, or may be a standard strain or an actual clinical sample.

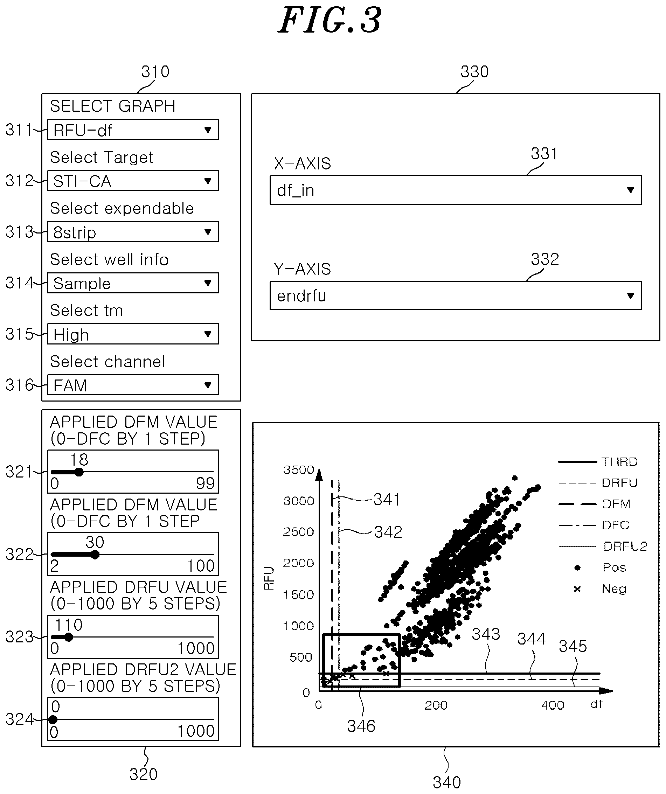

According to one embodiment, the correct answer data may be displayed in a position corresponding to the coordinate system displayed on the interface, with the positive and negative being distinguished by colors or icons, which will be described in detail later with reference to FIG. 3.

Meanwhile, the aforementioned parameter threshold-setting interface providing device 100 is for providing an interface for setting thresholds of various amplification characteristic parameters used as positive/negative determination criteria. According to one embodiment, the parameter threshold-setting interface providing device 100 may perform analysis for the presence/absence determination process for a target analyte using BPN (WO 2017/086762) and DSP (WO 2019/066572) technologies as positive/negative determination criteria. The parameters of BPN and DSP may be applied to the functions provided by the parameter threshold-setting interface providing device 100 for setting the value of the aforementioned Ct parameter.

For example, BPN is one of the methods for correcting and analyzing a data set. In BPN, a normalization coefficient for correcting a data set obtained from the aforementioned signal generation reaction for the target analyte using the signal generating means is provided, the normalization coefficient being provided using a data set including a plurality of data points including a reference value, a reference cycle and the cycles of the signal generating reaction and signal values in the cycles, the cycle is selected from the cycles of the data set, wherein the reference value is an arbitrarily determined value, and the normalization coefficient is provided by determining a relationship between the signal value of the cycle corresponding to the reference cycle in the data set and the reference value, and the analysis can be performed by applying the normalization coefficient to the signal values of the data set to obtain corrected signal values and providing a corrected data set.

In DSP, a data set for a target analyte is obtained from the signal generation reaction using the signal generating means to analyze the target analyte without false positive and false negative results, especially false positive results, using the fitting accuracy of a nonlinear function for the data set as a direct indicator for target analyte analysis, and by correcting the obtained data set including a plurality of data points including cycle numbers and signal values generating a nonlinear function for the corrected data set, the fitting accuracy of the nonlinear function for the corrected data set is determined, and the presence or absence of the target analyte in the sample can be determined using the fitting accuracy. This analysis method requires various parameters and can be optimized according to the values set for each parameter. By assigning parameters used in the presence/absence determination process for a target analyte using BPN (WO 2017/086762) and DSP (WO 2019/066572) technologies, an interface for setting the values of the BPN and DSP parameters can be provided.

Such parameters are used for mathematical processing of the signal in a signal generation reaction which generates a signal dependent on the presence of the target analyte, or as a criterion for determining the presence or absence of the target analyte in the sample. Examples of values which can determine the positive/negative of a sample include: Ct (Threshold Cycle), delta Ct, melt Tm, melt peak, threshold intensity value of the signal used for determining Ct, threshold for determining the Ct value; fitting start cycle and end cycle used for background subtraction; reference value and reference cycle when adopting the algorithm disclosed in WO 2017/086762; R2 value, maximum slope value of the sigmoid fitting curve and difference between maximum signal value and minimum signal value when using a sigmoid fitting algorithm; reference values when adopting the algorithms disclosed in WO 2015-147370, WO 2015/147412, WO 2015/147382 and WO 2016/093619.

Accordingly, the BPN analysis method may include parameters for a reference value (RV), a signal value relationship parameter (cut-off ratio (CR)) value. The CR describes, in particular numerically, a signal change, the relationship or degree of the signal change or signal difference when two signal values occur at different detection temperatures. In this case, the different detection temperatures may be a relative high detection temperature and a relative low detection temperature, or a first temperature and a second temperature. The "signal value relationship parameter (CR)" includes any value that reflects a pattern (rule) of the signal change at different detection temperatures. In addition, the signal value relationship parameter refers to a value representing the degree of change between a signal detected at the relative high detection temperature and a signal detected at the relative low detection temperature for a specific target nucleic acid sequence. The signal value relationship parameter may refer to a value used to convert, switch, adjust, or transform a signal detected at one temperature into a signal at another temperature. The signal value relationship parameter may vary depending on the type of the target nucleic acid sequence, the type of the signal generating means, and the conditions of incubation and detection. Therefore, various signal value relationship parameters can be determined for different or identical target nucleic acid sequences.

The signal value relationship parameters can be represented in various forms. For example, the signal value relationship parameters can be represented as numerical values, the presence/absence of a signal, or a plot with signal characteristics.

The DSP includes parameters for correction and parameters for numerical adjustment for a data set obtained through a signal generation reaction. The parameters for correction for the data set may include a PMC (Parameter Criterion) for determining the shape of a sigmoid graph for the data set obtained through the signal generation reaction, an SFC (Start Fitting Cycle) for correction of a start cycle for baseline fitting, and an MFC (Minimum Fitting Cycle) for correcting the data set by using the baseline fitting from the SFC to the MFC at least.

The parameters for numerical adjustment may include a CR (Cut-off ratio) which is a value obtained by dividing an RFU value at a low temperature by an RFU value at a high temperature to correct the difference in REU value due to the difference in RFU values of Low/High, a threshold (THRD, Ct Threshold) for determining positive/negative of a sample after sigmoid fitting, DRFU (Determinant RFU) for filtering positive/negative of a sample, and RC (R Square Criterion) which indicates negative processing if R^2 is lower than or equal to R Square Criterion. In addition, U.S. Patent Application Publication No. 2009/0119020 discloses that whether a data set shows significant or valid growth can be determined using R^2 value of a sigmoid function, and in this case, the parameters for numerical adjustment may further include a parameter RC which indicates negative if the R^2 value is lower than or equal to R Square Criterion. These parameters may have values set for each detection channel (FAM, HEX, C610, Q670, Q705) and each detection temperature.

The parameter values according to these types of analysis methods are values for determining positive/negative to converge on the positive/negative determination results confirmed in clinical trials.

As described above, the interface providing unit 180 may display the results in various ways when the data generated from the reaction well where the nucleic acid amplification reaction has been performed on the plate to be analyzed is processed by applying BPN and DSP technologies.

FIG. 3 illustrates a parameter setting interface for performing a parameter threshold-setting interface providing method according to one embodiment.

Referring to FIG. 3, the parameter threshold-setting interface providing device 100 provides a display 300 on which a determination process setting section 310, an amplification characteristic parameter threshold setting section 320, an axis setting section 330, and an amplification characteristic parameter display section 340 are displayed through the interface providing unit 180.

The parameter threshold-setting interface providing device 100 may select a sample to be read and set items related to the determination process through the determination process setting section 310. The determination process setting section 310 may include a graph selection section 311 for selecting the type of graph to be displayed, a target selection section 312 for selecting the type of target to be analyzed, a consumable selection section 313 for selecting the type of container, a well information selection section 314 for selecting a well type such as sample/positive control/negative control, a temperature selection section 315 for selecting a detection temperature, and a channel selection section 316 for selecting a detection channel.

Hereinafter, subsections and their functions that may be included in the determination process setting section 310.

In one embodiment, the parameter threshold-setting interface providing device 100 may display measurement values for each of a plurality of samples by using visual means through the selected graph. The measurement values are values obtained by measuring amplification characteristic parameters that reflect the characteristics of the amplification curve described above for detection data obtained during the detection process for the target analyte for each of the plurality of samples. The amplification characteristic parameters may be parameters used in the presence/absence determination process for the target analyte using the DSP technology described above.

For example, the graph may be a two-dimensional planar graph, where the X and Y axes respectively relate to df and RFU, or to α4 and df. As disclosed in the aforementioned WO 2019/066572, df is the maximum slope of a nonlinear function fitted to a data set or a standardized data set, RFU (relative fluorescence unit) is a signal value, and α4 is one of the parameters (α1, α2, α3, α4) of the nonlinear function.

Each of the plurality of samples may undergo a data processing process, e.g., BPN or DSP, from the detection data obtained during the detection process for the target analyte, and as a result, the aforementioned types of parameters may be derived. The derived parameters may be displayed on a coordinate system to correspond to the parameters of the axes of the selected graph.

As described above, the parameter threshold-setting interface providing device 100 may display each of the derived values, i.e., measurement values, for specific amplification characteristic parameters for a plurality of samples on a coordinate space through the interface providing unit 180.

The target selection section 312 may be included in the determination process setting section 310 to allow a user to select the type of target to be analyzed. The target nucleic acid sequence includes any naturally occurring prokaryotic nucleic acid, eukaryotic (e.g., protozoa and parasites, fungi, yeast, higher plants, lower animals, and higher animals including mammals and humans) nucleic acid, viral (e.g., herpes virus, HIV, influenza virus, Epstein-Barr virus, hepatitis virus, poliovirus, etc.) nucleic acid, and viroid nucleic acid. The target nucleic acid sequence includes a nucleic acid molecule that is produced or can be produced by a recombinant method or a chemical synthetic method. Accordingly, the target nucleic acid sequence may or may not exist in nature. The target nucleic acid sequence includes known or unknown sequences.

As described above, according to one embodiment, the parameter threshold-setting interface providing device 100 can provide a means for setting thresholds using data for a specific target selected through the target selection section 312.

Meanwhile, as the reaction vessel, various types of vessels, such as test tubes, PCR tubes, strip tubes, and multi-well PCR plates, may be used. Such vessels are generally consumables, and the resulting signal pattern may vary depending on the type of vessel. Accordingly, setting parameters according to the vessel used for the reaction may be considered a necessary configuration for accurate determination. According to one embodiment, the parameter threshold-setting interface providing device 100 may provide the consumable selection section 313 for selecting the type of reaction vessel.

Meanwhile, the wells used in the determination process may contain samples for analysis, or positive or negative controls to confirm whether the experiment is performed correctly or not. The well information selection section 314 may include well information to enable selection of well information such as a sample, a positive control, or a negative control.

Meanwhile, data sets for target analytes may be measured at different temperatures. In this regard, the subject applicant has developed the MuDT technology (WO 2015/147412) for detecting multiple target nucleic acid sequences using a single type of detector in a single reaction vessel. In the MuDT technology, the presence of a target nucleic acid sequence having a relative low detection temperature is determined by (i) a signal detected at both a relative high detection temperature and a relative low detection temperature, and (ii) a reference value representing the relationship of signal changes between different detection temperatures. When a first target nucleic acid sequence is a target nucleic acid sequence having the relative high detection temperature and a second target nucleic acid sequence is a target nucleic acid sequence having the relative low detection temperature, a signal for the first target nucleic acid sequence is removed using the reference value for the first target nucleic acid sequence to extract a signal for the second target nucleic acid sequence from the signal detected at the relative low detection temperature. That is, data can be obtained at each of the relative low detection temperature and the relative high detection temperature for the target analyte, and each data can be selected through the temperature selection section 315.

As described above, according to one embodiment, the parameter threshold-setting interface providing device 100 can select data detected at a relatively high or low temperature through the temperature selection section 315.

In the above, the processor setting that is performed through the determination process setting section 310 has been described. Through the determination process setting section 310, the parameter threshold-setting interface providing device 100 according to one embodiment can select data required to perform the determination process and determine a means for confirming the result produced through the determination process.

According to one embodiment, the parameter threshold-setting interface providing device 100 may set thresholds of amplification characteristic parameters through the amplification characteristic parameter threshold setting section 320. The amplification characteristic parameters are parameters extracted from an amplification curve approximated from detection data obtained during the detection process for each target analyte in the sample. The thresholds of the amplification characteristic parameters are values that can serve as a boundary between positive and negative when determining whether the sample is positive or negative using the amplification characteristic parameters.

According to one embodiment, the parameter threshold-setting interface providing device 100 may include a dfM setting bar 321 for setting dfM, a dfC setting bar 322 for setting dfC, a DRFU setting bar 323 for setting DRFU, and a DRFU2 setting bar 324 for setting DRFU2. In FIG. 3, the amplification characteristic parameter threshold setting section 320 is implemented by using an adjustment bar as an example, but various means for adjusting or inputting numerical values may be applied as a substitute in the amplification characteristic parameter threshold setting section 320.

First, dfM and dfC are parameters related to the aforementioned df. As described above, df is the maximum slope of a nonlinear function fitted to a data set or a standardized data set. In this case, if the measured df for a specific sample is greater than or equal to dfC, the sample can be read as positive. Also, if the measured df for a specific sample is less than dfM, the sample can be read as negative. Meanwhile, if df is greater than or equal to dfM and less than dfC, other amplification characteristic parameters, e.g., α4, which is a parameter related to the amplification efficiency of the sigmoid function, may be used to determine whether the sample is positive or negative.

According to one embodiment, the parameter threshold-setting interface providing device 100 may perform negative filtering by setting dfM, which is a minimum threshold for df, using the dfM setting bar 321, and may perform positive filtering by setting dfC, which is a maximum threshold for df, using the dfC setting bar 322. This shows that there can be three or more states caused by thresholds, not two, and according to one embodiment, the dfM and dfC settings using the parameter threshold-setting interface providing device 100 can allow determination of a third state in addition to positive and negative. For example, the third state may be a gray zone for which immediate determination of positive and negative is difficult.

Meanwhile, DRFU is a parameter related to the aforementioned RFU. If the difference between the signal value in the last cycle and the minimum signal value among the detection signals obtained from the sample does not exceed a threshold, the sample can be filtered as negative, and the threshold that can be used in this case may be DRUF.

According to one embodiment, the parameter threshold-setting interface providing device 100 may filter, as negative, a sample in which the difference between the signal value in the last cycle and the minimum signal value does not exceed the threshold, i.e., the amount of change in signal intensity is not large, by using the DRFU setting bar 323.

Meanwhile, DRFU2 is also a parameter related to the aforementioned RFU, and the aforementioned MuDT technology will be briefly reviewed for the explanation of DRFU2. A data set for a target analyte can be measured at different temperatures of a relative high temperature and a relative low temperature, and if a first target nucleic acid sequence is a target nucleic acid sequence having the relative high temperature detection temperature and a second target nucleic acid sequence is a target nucleic acid sequence having the relative low temperature detection temperature, a signal for the second target nucleic acid sequence can be extracted by removing a signal for the first target nucleic acid sequence from a signal detected at the relative low temperature detection temperature using the reference value for the first target nucleic acid sequence. In this case, if the second target nucleic acid sequence does not exist, the signals detected at the relative high and low temperatures are signals for the first target nucleic acid sequence, so the signal detected after removing the signal for the first target nucleic acid sequence at the relative low temperature detection temperature using the reference value for the first target nucleic acid sequence should theoretically be "0". However, if the reference value setting does not accurately reflect the change in the measurement value due to the temperature change, even when the signal for the first target nucleic acid sequence is removed from the relative low temperature detection temperature using the reference value for the first target nucleic acid sequence, the signal may not become "0" and may remain. If the remaining signal undergoes a process such as BPN or DSP, for example, the resulting value may be displayed on the parameter threshold-setting interface providing device 100. In this case, by using the DRFU2 setting bar, an additional threshold related to RFU other than DRFU can be introduced, thereby filtering out processing errors for various cases such as the reference value setting error as described above.

The types and functions of thresholds set in the amplification characteristic parameter threshold setting section 320 have been described above. Meanwhile, although not shown in the drawings, the parameter threshold-setting interface providing device 100 according to one embodiment may use other parameters for determining the positive or negative of a sample. For example, there are parameters used for mathematical processing of a signal in a signal generation reaction that generates a signal dependent on the presence of a target analyte or used as a criterion for determining the presence or absence of the target analyte in the sample. Examples of values that can be used to determine the positive/negative of the sample include Ct (Threshold Cycle), delta Ct, melt Tm, melt peak, threshold intensity value of the signal used for determining Ct, threshold for determining the Ct value; fitting start cycle and end cycle used for background subtraction; reference value and reference cycle when adopting the algorithm disclosed in WO 2017/086762; R2 value, maximum slope value of the sigmoid fitting curve and difference between maximum signal value and minimum signal value when using a sigmoid fitting algorithm; and the reference value when adopting the algorithms disclosed in WO 2015-147370, WO 2015/147412, WO 2015/147382, and WO 2016/093619.

Accordingly, the BPN analysis method may include parameters for a reference value (RV), a signal value relationship parameter (cut-off ratio (CR)) value. The CR describes, in particular numerically, a signal change, the relationship or degree of the signal change or signal difference when two signal values occur at different detection temperatures. In this case, the different detection temperatures may be a relative high detection temperature and a relative low detection temperature, or a first temperature and a second temperature. The "signal value relationship parameter (CR)" includes any value that reflects a pattern (rule) of the signal change at different detection temperatures. In addition, the signal value relationship parameter refers to a value representing the degree of change between a signal detected at the relative high detection temperature and a signal detected at the relative low detection temperature for a specific target nucleic acid sequence. The signal value relationship parameter may refer to a value used to convert, switch, adjust, or transform a signal detected at one temperature into a signal at another temperature. The signal value relationship parameter may vary depending on the type of the target nucleic acid sequence, the type of the signal generating means, and the conditions of incubation and detection. Therefore, various signal value relationship parameters can be determined for different or identical target nucleic acid sequences.

The signal value relationship parameters can be represented in various forms. For example, the signal value relationship parameters can be represented as numerical values, the presence/absence of a signal, or a plot with signal characteristics.

The DSP includes parameters for correction and parameters for numerical adjustment for a data set obtained through a signal generation reaction. The parameters for correction for the data set may include a PMC (Parameter Criterion) for determining the shape of a sigmoid graph for the data set obtained through the signal generation reaction, an SFC (Start Fitting Cycle) for correction of a start cycle for baseline fitting, and an MFC (Minimum Fitting Cycle) for correcting the data set by using the baseline fitting from the SFC to the MFC at least.

The parameters for numerical adjustment may include a CR (Cut-off ratio) which is a value obtained by dividing an RFU value at a low temperature by an RFU value at a high temperature to correct the difference in REU value due to the difference in RFU values of Low/High, a threshold (THRD, Ct Threshold) for determining positive/negative of a sample after sigmoid fitting, dRFU (Determinant RFU) for filtering positive/negative of a sample, and RC (R Square Criterion) for processing samples as negative if R^2 is lower than or equal to R Square Criterion. In addition, U.S. Patent Application Publication No. 2009/0119020 discloses that whether a data set shows significant or valid growth can be determined using R2 value of a sigmoid function, and in this case, the parameters for numerical adjustment may further include a parameter RC which indicates negative if the R2 value is lower than or equal to R Square Criterion. These parameters may have values set for each detection channel (FAM, HEX, C610, Q670, Q705) and each detection temperature.

As described above, through the threshold setting section 320, the parameter threshold-setting interface providing device 100 according to one embodiment can set the threshold of parameters that can be criteria for determining the positive or negative of a sample.

Next, the axis setting section 330 may set parameters corresponding to the axis. The axis setting section 330 may select parameters not presented in the aforementioned graph selection section 311, for example. In addition to the aforementioned DRFU and df, parameters that can be presented through the axis setting section 330 may also include other parameters related to the sigmoid function, such as α4, for example.

The axis setting section 330 may further include sections related to each axis of the coordinate system therein. Referring to FIG. 3, the axis setting section 330 may re-select detailed parameters related to the axis of the graph selected in the graph selection section 311 of the determination process setting section 310 through an X-axis parameter setting section 331 and a Y-axis parameter setting section 332.

Meanwhile, according to one embodiment, the parameter threshold-setting interface providing device 100 may set parameters for each axis through the axis setting section 330 without providing the graph selection section 311.

According to one embodiment, the display 300 may provide the amplification characteristic parameter display section 340. An arbitrary coordinate system may be displayed on the display section 340. In the arbitrary coordinate system, measurement values for a plurality of amplification characteristic parameters, which are measured from detection data obtained during a detection process for a target analyte in each of a plurality of samples, may be displayed at corresponding positions of the coordinate system. The display section 340 of FIG. 3 displays a two-dimensional coordinate system, and on the coordinate system, a positive sample is indicated by a circle (O) symbol, and a negative sample is indicated by a cross (X) symbol. The X-axis of the coordinate system is an axis related to df, which is the maximum slope of a nonlinear function fitted to a data set or a standardized data set, and the Y-axis of the coordinate system is an axis related to RFU (relative fluorescence unit), which is a signal value. As described above, a plurality of thresholds may exist for parameters corresponding to each axis, and these thresholds may be displayed on the coordinate system. Parameters related to RFU, which correspond to the Y-axis, may include THRD, DRFU, and DRFU2, and corresponding boundary lines 343, 344, and 345 may be displayed on the coordinate system, respectively. Parameters related to df, which correspond to the X-axis, may include DFM and DFC, and corresponding boundary lines 341 and 342 may be displayed on the coordinate system, respectively. Meanwhile, the boundary lines 341, 342, 343, 344, and 345 are displayed as straight lines parallel to the axes on the drawing, but when the displayed parameter is related to a parameter of the other axis, i.e., is not independent of other parameters, its boundary line may be displayed as a straight line not parallel to the axis or a curved line.

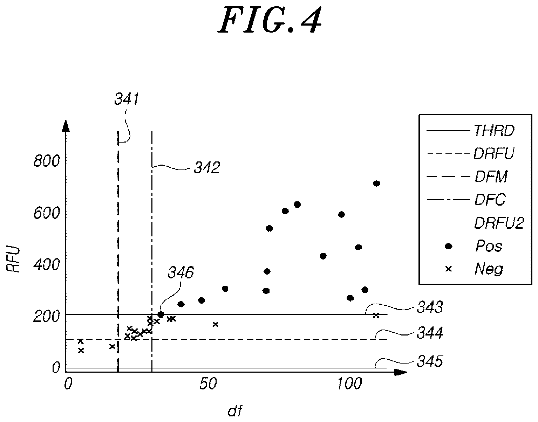

Meanwhile, when the amplification characteristic parameter threshold is changed through the amplification characteristic parameter threshold setting section 320 described above, the boundary lines 341, 342, 343, 344, and 345 on the coordinate system displayed in the amplification characteristic parameter display section 340 may be shifted in position and displayed to match the changed value on the coordinate system. According to one embodiment, the parameter threshold-setting interface providing device 100 may measure the amplification characteristic parameter for each sample by using a sample that is pre-labeled as either a positive sample or a negative sample. When the measured amplification characteristic parameter is displayed at a corresponding position in the aforementioned coordinate system, the positive samples and the negative samples may be grouped as indicated on the coordinate system of the display section 340 in FIG. 3. This is due to the difference in detection data obtained from each of the positive samples and the negative samples. If the boundaries of the groups can be determined by grouping positive and negative samples on the coordinate system in this way, these boundaries can be used to determine positive or negative of samples that are not labeled as positive or negative, that is, samples whose positive or negative has not been confirmed. Accordingly, an example of positive/negative determination using boundary lines 341, 342, 343, 344, and 345 indicating thresholds will be described with reference to FIG. 4.

Meanwhile, in one embodiment, the coordinate system displayed in the amplification characteristic parameter display section 340 may be a three-dimensional coordinate system (not shown). The displayed three-dimensional coordinate system may be, for example, a three-dimensional coordinate system in which a new axis is introduced in the aforementioned RFU-df two-dimensional plane graph. The parameters described in the present specification may be applied to the new axis without limitation. For example, a graph in which an R^2 axis is added to the aforementioned RFU-df two-dimensional plane graph will be described. As described above, R^2 is a parameter used to determine whether a data set of a sigmoid function shows significant or valid growth. Accordingly, the three-dimensional coordinate system in which the R^2 axis is added to the RFU-df graph can present a more sophisticated criterion for positive/negative determination by additionally showing the degree of validity of the data set with respect to the sample distribution.

FIG. 4 shows an enlarged view of a coordinate system region 346 in FIG. 3. First, it can be seen that the THRD boundary line 346 is a boundary line that can be used to confirm positive/negative determination using RFU. According to one embodiment, the parameter threshold-setting interface providing device 100 can set criteria for future positive/negative determination by using pre-labeled positive/negative samples. For example, the THRD boundary line 346 of FIG. 4 may be one of the criteria. Using a sample 346 touching the THRD boundary line 346 as a reference, it can be seen that all samples having an RFU greater than the sample 346 are positive, which can be used as a criterion for future positive/negative determination. In addition, in the case of the DRFU boundary line 344, the DRFU can be used as a negative filter before the aforementioned DSP process, in which case samples below the DRFU boundary line 344 should theoretically not be detected. Therefore, depending on whether DRFU was used as a negative filter before DSP, the DRFU boundary line 344 can be utilized as an indicator requiring additional interpretation. As can be seen from FIG. 4, the DRFU2 boundary line 345 is set to "0" and is not utilized in the analysis of the current samples.

Meanwhile, the dfM boundary line 341 and the dfC boundary line 342, which are boundary lines related to df, respectively represent the lower and upper limits for determining positive and negative based on df. As described above, df is the maximum slope of a nonlinear function fitted to a data set or a standardized data set, and it can be an indicator of negative if its value is less than the lower limit, and an indicator of positive if its value is greater than the upper limit. If the df value is between the upper and lower limits, other parameters can be used to determine positive or negative. Meanwhile, it can be seen through the distribution of the sample values in FIG. 4 that if the RFU value is less than THRD, samples are negative even when the df value is greater than or equal to dfC.

As described above, according to one embodiment, the parameter threshold-setting interface providing device 100 can provide an interface for visually confirming parameters that can be used as criteria for determining positive or negative in the positive/negative determination process. In addition, the parameter threshold-setting interface providing device 100 can provide an interface in which the parameters can be represented as vector values on a coordinate system of two or more dimensions to enable effective visualization of their distribution and the corresponding boundary value setting.

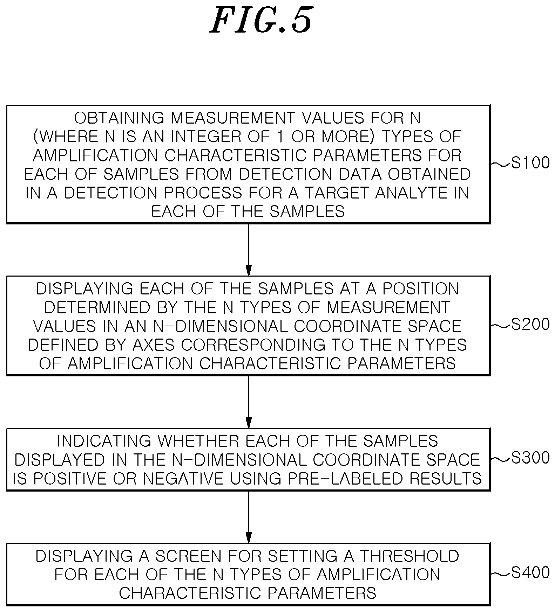

FIG. 5 is a flowchart of a method for providing an interface for setting a threshold of an amplification characteristic parameter according to one embodiment. It is noted, however, that FIG. 5 is merely illustrative, and the idea of the present disclosure is not limited to what is illustrated in FIG. 5. Referring to FIG. 5, a step of obtaining measurement values for n (where n is an integer of 2 or more) types of amplification characteristic parameters for each of a plurality of samples from detection data obtained in a detection process for a target analyte in each of the plurality of samples is performed (S100). At this step, each of the plurality of samples may be pre-labeled as either a positive sample or a negative sample. In this case, the detection process may be performed by a nucleic acid amplification reaction, an enzyme reaction, or a microbial growth. In addition, the n types of amplification characteristic parameters may include at least one of a degree of matching between an approximation function approximating the detection data and the detection data, a graph shape parameter representing a graph shape of the approximation function, and a derived parameter derived from the graph shape parameter. In this case, the approximation function may be a function having the sigmoid graph profile as described above. In addition, the graph shape parameter of the approximate function or the derived parameter may include at least one of the maximum value of the first derivative of the approximate function, the difference between the maximum signal value of the approximate function and the background signal value, and the sharpness of the approximate function. In addition, the positive/negative determination process may include a process of comparing a measurement value of at least one parameter among the matching degree, the maximum value of the first derivative, the difference, and the sharpness of the approximate function with a corresponding threshold.

Next, a step of displaying each of the plurality of samples at a position determined by the n types of measurement values in an n-dimensional coordinate space defined by axes corresponding to the n types of amplification characteristic parameters is performed (S200). In this case, the axes corresponding to the n types of amplification characteristic parameters in the n-dimensional coordinate space may be orthogonal to each other. In addition, at least one of the parameters corresponding to each axis of the coordinate system may be selected by the user.

Next, a step of indicating whether each of the plurality of samples displayed in the n-dimensional coordinate space is positive or negative is performed using the pre-labeled results (S300). In this case, the step of indicating whether each of the plurality of samples is positive or negative may indicate the positive samples and the negative samples using different colors and/or shapes, respectively.

Next, a step of displaying a screen for setting a threshold for each of the n types of amplification characteristic parameters is performed (S400). In this case, the screen for setting the threshold includes a slide bar implemented to be adjustable on the screen for each of the n types of parameters, and the n types of thresholds are determined by adjusting the corresponding slide bar, and the threshold determined each time the slide bar is adjusted can be reflected and displayed in real time in the n-dimensional coordinate system space.

In this case, the setting screen may be displayed on one screen along with the n-dimensional coordinate system space in which each of the plurality of samples is labeled as positive or negative. According to one embodiment, the setting screen may be the determination process setting section 310, the amplification characteristic parameter threshold setting section 320, and the axis setting section 330.

Further, each of the n types of thresholds may be used as a criterion for determining positive or negative in the positive/negative determination process for each of the plurality of samples, and the thresholds may be represented as lines or planes in the coordinate space. In addition, the lines or planes may be used to group the plurality of samples displayed in the coordinate space into a positive sample group and a negative sample group.

Meanwhile, the thresholds may include an upper threshold and a lower threshold. In this case, a step of indicating a sample located between the upper threshold and the lower threshold as a third state result other than the pre-labeled results may be further included. For example, the third state result may be a gray zone.

Hereinafter, the method for providing an interface for setting the threshold of such amplification characteristic parameter is performed in the parameter threshold-setting interface providing device 100. For specific details on the method, the description of the parameter threshold-setting interface providing device 100 will be referred to.

In addition, the terms "include," "comprise," or "have" described above, unless otherwise specifically stated, mean that the corresponding component can be included, and therefore should be interpreted as including other components rather than excluding other components. All terms, including technical or scientific terms, unless otherwise defined, have the same meaning as commonly understood by a person of ordinary skill in the art to which the present disclosure pertains. Commonly used terms, such as terms defined in a dictionary, should be interpreted as being consistent with the contextual meaning of the relevant technology, and should not be construed in an idealized or unduly formal sense, unless explicitly defined in the present disclosure.

The above-described embodiments are merely examples, and it will be appreciated by one of ordinary skill in the art various changes may be made thereto without departing from the scope of the present disclosure. Accordingly, the embodiments set forth herein are provided for illustrative purposes, but not to limit the scope of the present disclosure, and should be appreciated that the scope of the present disclosure is not limited by the embodiments. The scope of the present disclosure should be construed by the following claims, and all technical spirits within equivalents thereof should be interpreted to belong to the scope of the present disclosure.

While embodiments of the present disclosure have been described above, it will be apparent to one of ordinary skill in the art that the specific techniques are merely preferred embodiments and the scope of the present disclosure is not limited thereto. Thus, the scope of the present disclosure is defined by the appended claims and equivalents thereof.

### CROSS-REFERENCE TO RELATED APPLICATION

This patent application claims priority, under 35 U.S.C. §119(a), to the Korean patent application No. 10-2022-0094697, filed on July 29, 2022, the entire contents of which are incorporated herein by reference. Furthermore, this patent application also claims priority to countries other than the United States for the same reasons, the entire contents of which are incorporated herein by reference.

## Claims

1. A method for providing an interface for setting positive/negative determination criteria for amplification characteristic parameters, the method comprising:
obtaining measurement values for n types of amplification characteristic parameters for each of a plurality of samples from detection data obtained in a detection process for a target analyte in each of the plurality of samples, wherein n is an integer of 2 or more and each of the plurality of samples is pre-labeled as either a positive sample or a negative sample;
displaying each of the plurality of samples at a position determined by the n types of measurement values in an n-dimensional coordinate system space defined by axes corresponding to the n types of amplification characteristic parameters;
indicating whether each of the plurality of samples displayed in the n-dimensional coordinate system space is positive or negative using the pre-labeled results; and
displaying a screen for setting a threshold for at least one of the n types of amplification characteristic parameters,
wherein the threshold is used as a criterion in a positive/negative determination process for each of the plurality of samples.

2. The method of claim 1, wherein the screen for setting the threshold value is displayed simultaneously with the n-dimensional coordinate system space.

3. The method of claim 1, wherein the detection process is performed by nucleic acid amplification reaction, enzymatic reaction, or microbial growth.

4. The method of claim 1, wherein the n types of amplification characteristic parameters include at least one of (1) a matching parameter indicating a degree of matching between the detection data and an approximation function approximating the detection data, (2) a graph shape parameter representing a graph shape of the approximation function, and (3) a derived parameter derived from the graph shape parameter.

5. The method of claim 4, wherein the n types of amplification characteristic parameters include at least one of a maximum value of the first derivative of the approximation function, a difference between a maximum signal value and a minimum signal value of the approximation function, or a sharpness of the approximation function.

6. The method of claim 5, wherein in the positive/negative determination process, a measurement value of at least one of the n types of amplification characteristic parameters and the threshold value corresponding thereto are compared.

7. The method of claim 1, wherein in the n-dimensional coordinate system space, the axes corresponding to the n types of amplification characteristic parameters are orthogonal to each other.

8. The method of claim 1, wherein the positive sample and the negative sample are indicated using different colors and/or shapes.

9. The method of claim 1, wherein at least one of the parameters corresponding to the axes of the n-dimensional coordinate system is selected by a user.

10. The method of claim 1, wherein the screen for setting the threshold value is provided with a slide bar that is implemented to be adjustable on the screen,
the threshold value is determined by adjusting the corresponding slide bar, and
the threshold value determined each time the slide bar is adjusted is reflected and displayed in real time in the n-dimensional coordinate system space.

11. The method of claim 1, wherein the threshold value is represented as a line or plane in the n-dimensional coordinate system space.

12. The method of claim 11, wherein the line or plane is used to group the plurality of samples displayed in the n-dimensional coordinate system space into a positive sample group and a negative sample group.

13. The method of claim 1, wherein the threshold value includes an upper threshold value and a lower threshold value.

14. The method of claim 13, further comprising:
indicating at least one of the samples located between the upper threshold value and the lower threshold value as a third state other than the pre-labeled results.

15. A computer program stored on a computer-readable recording medium, which is programmed to perform each step included in the method according to any one of claims 1 to 14.

16. A computer-readable recording medium storing a computer program programmed to perform each step included in the method according to claim 15.

17. A device for providing an interface for setting positive/negative determination criteria for amplification characteristic parameters of a target analyte, the device comprising:
a memory storing at least one instruction; and
a processor,
wherein the processor executes the at least one instruction to perform:
obtaining measurement values for n types of amplification characteristic parameters for each of a plurality of samples from detection data obtained in a detection process for a target analyte in each of the plurality of samples, wherein n is an integer of 2 or more and each of the plurality of samples is pre-labeled as either a positive sample or a negative sample;
displaying each of the plurality of samples at a position determined by the n types of measurement values in an n-dimensional coordinate system space formed by axes corresponding to the n types of amplification characteristic parameters;
indicating whether each of the plurality of samples displayed in the n-dimensional coordinate system space is positive or negative using the pre-labeled results; and
displaying a screen for setting a threshold for at least one of the n types of amplification characteristic parameters,
wherein each of the n types of thresholds is used as a criterion in a positive/negative determination process for each of the plurality of samples.
